## Europäisches Patentamt
## European Patent Office
## Office européen des brevets

(11) Veröffentlichungsnummer: **0 252 250**
**A2**

(12) **EUROPÄISCHE PATENTANMELDUNG**

(21) Anmeldenummer: **87106974.6**

(22) Anmeldetag: **14.05.87**

(51) Int. Cl.⁴: **C07C 69/33** , C07C 67/08

(30) Priorität: **11.07.86 DE 3623371**

(43) Veröffentlichungstag der Anmeldung:
**13.01.88 Patentblatt 88/02**

(84) Benannte Vertragsstaaten:
**AT BE CH DE ES FR GB IT LI NL SE**

(71) Anmelder: **HÜLS AKTIENGESELLSCHAFT**
**Patentabteilung / PB 15 - Postfach 13 20**
**D-4370 Marl 1(DE)**

(72) Erfinder: **Bickert, Peter, Dr.**
**318 Westgate Dr.**
**North Edison New Jersey 08820(US)**

(54) **Kohlenhydrat-Fettsäureester und ein Verfahren zu ihrer Herstellung.**

(57) Die Erfindung betrifft Kohlenhydrat-Fettsäureester mit verbesserter Farbe und Fließfähigkeit und ein einstufiges Verfahren zur ihrer Herstellung. Zur Herstellung der Produkte werden Kohlenhydrate mit Fettsäuren in Gegenwart von Polyetherpolyolen verestert.

EP 0 252 250 A2

## Kohlenhydrat-Fettsäureester und ein Verfahren zu ihrer Herstellung

Die Erfindung betrifft Kohlenhydrat-Fettsäureester, die hinsichtlich Farbe und Handhabbarkeit verbessert sind, sowie ein einstufiges Verfahren zu ihrer Herstellung.

Kohlenhydrat-Fettsäureester und Verfahren zur ihrer Herstellung sind bekannt. Sie können nach EP 0 132 293 und EP 0 132 941 durch Umesterung von Fettsäureestern kurzkettiger Alkohole mit einem Saccharid oder einem Zuckeralkohol hergestellt werden. Darüber hinaus sind auch Verfahren bekannt, bei denen eine direkte Veresterung von Kohlenhydraten mit freien Fettsäuren durchgeführt wird. Durch direkte Veresterung werden nach US-PS 4 297 290 Sorbitanfettsäureester hergestellt. Dabei stellt man zunächst aus Sorbitol säurekatalysiert Anhydrosorbitol her, das dann basenkatalysiert mit einer Fettsäure umgesetzt wird. Die Farbe der Produkte wird durch Aktivkohle, Phosphorsäure und Diatomeenerde verbessert.

Es werden also entweder eine Veresterung und eine Umesterung oder eine Anhydridbildung und eine Veresterung durchgeführt. Die genannten Verfahren erfordern demnach mindestens 2 Reaktionsstufen, wobei eine ggf. durchgeführte Aktivkohlebehandlung besonders bei hochviskosen Produkten eine weitere, verfahrenstechnisch schwierige Stufe darstellt.

Nach DE-OS 31 19 553 werden Carbonsäureester von Anhydrohexiten hergestellt, indem man Hexite, vorzugsweise Sorbit, mit Carbonsäuren unter Alkalikatalyse bei 210 bis 260 °C umsetzt und die Produkte anschließend mit Wasserstoffperoxid behandelt.

Dieses einstufige Verfahren liefert jedoch, vor allem wenn man partiell hydrierte Stärkehydrolysate mit Fettsäuren umsetzt, Produkte, die für den Einsatz als Emulgatoren in hochwertigen Kosmetika oder Nahrungsmitteln hinsichtlich Klarheit, Farbe und Fließfähigkeit noch nicht voll befriedigen.

Aufgabe der vorliegenden Erfindung ist es, ein einfaches Verfahren zur Herstellung von Kohlenhydrat-Fettsäureestern von guter Farbqualität und geringem Trübungsgrad zu entwickeln. Eine weitere Aufgabe besteht darin, die Handhabbarkeit der Kohlenhydrat-Fettsäureester durch Erhöhung ihrer Fließfähigkeit zu verbessern.

Überraschend wurde nun gefunden, daß man farblich verbesserte Kohlenhydrat-Fettsäureester mit verminderter Konsistenz erhält, wenn man dem Veresterungsgemisch aus Kohlenhydrat und Fettsäure 1 bis 15 Gew.-% Polyetherpolyol, bezogen auf die Gesamtmenge der Reaktionskomponenten, sowie eine wirksame Menge Hypophosphit als Farbverbesserer zusetzt und nach beendeter Reaktion das Reaktionsgemisch mit Wasserstoffperoxid bleicht.

Geeignete Polyetherpolyole sind Polyethylenglykol, Polypropylenglykol oder Polyglycerin. Vorzugsweise werden Polyglycerine mit mittleren Polymerisationsgraden von 2 bis 5 verwendet.

Monomeres Glycerin mit einem Polymerisationsgrad 1 hat nur einen geringen, nicht ausreichenden Effekt auf die Farbverbesserung und Viskositätserniedrigung.

Man kann auch Gemische von Polyglycerinen mit cyclischen Polyetherpolyolen, wie sie beispielsweise in DE-OS 21 33 281 beschrieben werden, einsetzen.

Vorzugsweise werden die Polyetherpolyole in Konzentrationen von 4 bis 10 Gew.-%, bezogen auf die Gesamtmenge der Reaktionskomponenten, verwendet.

Die Polyetherpolyole werden den Ausgangskomponenten zugemischt oder während der Veresterungsreaktion zugegeben.

Geeignete Kohlenhydrate für die Veresterung sind Zuckeralkohole mit 5 oder 6 C-Atomen. Beispiele für Hexite sind Sorbit, Mannit oder Dulcit. Geeignete Pentite sind Arabit, Ribit, Xylit oder Lyxit.

Als Fettsäuren kommen Carbonsäuren mit 6 bis 30 C-Atomen in Betracht. Sie können auch Doppelbindungen in der Kohlenstoffkette enthalten. Beispiele sind Octansäure, Decansäure, Dodencansäure, Ölsäure, Palmitinsäure, Stearinsäure, Kokosnußölfettsäure und Rübölfettsäure.

Dabei besteht Kokosnußölfettsäure im wesentlichen aus einem Gemisch aus Capronsäure, Caprylsäure, Caprinsäure, Laurinsäure, Myristinsäure, Palmitinsäure, Palmitoleinsäure, Stearinsäure, Ölsäure, Elaidinsäure und Linolsäure.

Die Hauptbestandteile der Rübölfettsäure sind Caprylsäure, Caprinsäure, Laurinsäure, Myristinsäure, Palmitinsäure, Palmitoleinsäure, Stearinsäure, Ölsäure, Elaidinsäure, Linolsäure, Arachinsäure, Gadoleinsäure, Behensäure und Erucasäure.

Die Veresterungsreaktion wird bei 180 bis 250 °C durchgeführt. Vorzugsweise liegt die Reaktionstemperatur bei 190 bis 220 °C.

Die Reaktionszeit liegt im allgemeinen bei 2 bis 10 Stunden.

Geeignete Katalysatoren sind beispielsweise KOH, NaOH und Soda.

Ein Farbverbesserer wird während der Reaktion, vorzugsweise bereits am Anfang der Reaktion, zugesetzt. Geeignete Farbverbesserer sind Hypophosphite wie Calcium-oder Natriumhypophosphit oder hypophosphorige Säure. Sie werden in Konzentrationen von 0,1 bis 0,5 Gew.-%, bezogen auf die Gesamtmenge der eingesetzten Reaktionskomponenten, verwendet.

Als Bleichmittel dient im allgemeinen Wasserstoffperoxid, das nach der Reaktion bei Temperaturen von 70 bis 85 °C in einem Anteil von 0,1 bis 1,5 Gew.-%, bezogen auf die Gesamtmenge der eingesetzten Reaktionskomponenten, zugegeben wird.

Die erfindungsgemäßen Produkte zeichnen sich gegenüber Kohlenhydrat-Fettsäureestern, die nach bekannten Verfahren hergestellt wurden, durch eine hellere Farbe, geringere Trübung, geringere Säurezahl und durch eine verbesserte Fließfähigkeit aus.

Kohlenhydrat-Fettsäureester sind oberflächenaktive Verbindungen, die besonders als Emulgatoren im Kosmetik-und Nahrungsmittelbereich, aber auch darüber hinaus als Polymerisationshilfsmittel und Gleitmittel Verwendung finden. Ein besonderer Vorteil des erfindungsgemäßen Verfahrens besteht darin, daß sich nach Zusatz von Polyglycerin im Verlaufe der Reaktion Ester aus Polyglycerin und Fettsäuren bilden, die ebenfalls der Gruppe der oberflächenaktiven Verbindungen angehören und ihrerseits z. B. als Nahrungsmittelemulgatoren bereits bekannt sind.

### Beispiel 1

Ein 30 l-Rührkessel mit Destillationsaufsatz, Thermoelement und einer Stickstoffeinleitung am Kesselboden wird unter Rühren mit 10.79 kg (38.5 Mol) Rübölfettsäure, 0.14 kg (3.5 Mol) NaOH-Pulver, 4.56 kg (17,5 Mol) 70 %iger wäßriger Sorbit-Lösung, 0.71 kg (5 Gew.-%) Polyglycerin mit dem mittleren Polymerisationsgrad 3 und 0.037 kg (0.25 Gew.-%) Natriumhypophosphit beschickt. Unter Durchleiten eines Stickstoffstromes heizt man auf. Ab 100 °C destilliert Wasser ab. Nach Erreichen von 200 °C hält man bei dieser Temperatur, bis eine entnommene Probe eine Säurezahl SZ < 2 aufweist. Sodann wird die Temperatur auf 80 °C gesenkt und der Reaktionsansatz durch tropfenweise Zugabe von 87 g 30 %igem Wasserstoffperoxid innerhalb 30 min gebleicht. Schließlich rührt man 1 Stunde bei 85 °C nach und trägt das Produkt aus.

Die Produkteigenschaften sind in Tabelle 1 zusammengestellt.

### Beispiele 2 bis 7, Vergleichsbeispiele A bis G

Es wird wie in Beispiel 1 verfahren. Die dabei eingesetzten Stoffe und ihre Mengen sowie die Eigenschaften der resultierenden Kohlenhydrat-Fettsäureester sind in Tabelle 1 zusammengestellt.

Bei höhermolekularen Kohlenhydraten (Polyglucosylsorbitole) beziehen sich die angegebenen Mol-Verhältnisse auf die Fettsäuremenge pro Glucosyl-Einheit ohne Berücksichtigung der tatsächlichen Molekulargewichtsverteilung.

3

Tabelle 1

| Nr. | Edukte | | | | | | Bedingungen | | Produkte | | | | | Zustandsbeschreibung bei |
|---|---|---|---|---|---|---|---|---|---|---|---|---|---|---|
| | Kohlenhydrat | g | Fettsäure | g | Mol-Verh. | PG g | Temp. °C | Zeit h | FZ | SZ | EZ | OHZ | $H_2O$ % | Raumtemperatur |
| A | Sorbit (70 %) | 456 | Rüböl | 1 079 | 1 : 2,2 | - | 200 | 6 | 7 | 4 | 136 | 178 | 0.3 | trüb, fließfähig |
| 1 | Sorbit (70 %) | 456 | Rüböl | 1 079 | 1 : 2,2 | 70 | 200 | 6 | 4 | 4 | 133 | 231 | 0.3 | klar, gut fließfähig |
| B | Sorbit (70 %) | 456 | Kokos | 781 | 1 : 2,2 | - | 200 | 5 | 2 | 4 | 172 | 221 | 0.2 | fest |
| 2 | Sorbit (70 %) | 456 | Kokos | 781 | 1 : 2,2 | 56 | 200 | 5 | 2 | 4 | 167 | 277 | 0.4 | klar, gut fließfähig |
| C | Sorbit | 319 | Kokos | 781 | 1 : 2,2 | - | 200 | 5 | 2 | 3 | 172 | 212 | 0.2 | fest |
| 3 | Sorbit | 319 | Kokos | 781 | 1 : 2,2 | 56 | 200 | 4 | 2 | 6 | 167 | 286 | 0.3 | begrenzt fließfähig |
| D | Sorbit (70 %) | 456 | Kokos | 781 | 1 : 2,2 | - | 225 | 5 | 7 | 3 | 106 | 189 | 0.3 | fest, erst oberhalb 90 °C fließfähig |
| 4 | Sorbit (70 %) | 456 | Kokos | 781 | 1 : 2,2 | 56 | 225 | 3 | 4 | 4 | 172 | 222 | 0.1 | leicht flockig |
| E | M-Glucosid | 300 | Rüböl | 883 | 1 : 2,2 | - | 200 | 10 | 100 | 11 | 140 | 150 | 3.0 | leicht trüb, fließfähig, dunkel |
| 5 | M-Glucosid | 300 | Rüböl | 883 | 1 : 2,2 | 60 | 200 | 10 | 35 | 11 | 139 | 202 | 3.1 | klar, fließfähig |
| F | PGS III | 562 | Rüböl | 1 079 | 1 : 1,1 | - | 200 | 6 | 20 | 18 | 133 | 219 | 3.9 | trüb, fließfähig, Bodensatz |
| 6 | PGS III | 410 | Rüböl | 771 | 1 : 1,1 | 60 | 200 | 3 | 20 | 13 | 121 | 245 | 3.6 | klar, fließfähig, Bodensatz |
| G | PGS II | 1 396 | Rüböl | 668 | 4 : 1,1 | - | 200 | 3 | 80 | 14 | 71 | 627 | 7.8 | fest, gummiartig |
| 7 | PGS II | 1 047 | Rüböl | 501 | 4 : 1,1 | 78 | 200 | 3 | 80 | 12 | 73 | 450 | 12.3 | fließfähig |

0 252 250

Abkürzungen in der Tabelle:

FZ = Iodfarbzahl

SZ = Saurezahl     in mg KOH/g

EZ = Esterzahl     in mg KOH/g

OHZ = Hydroxylzahl in mg KOH/g

PG = Polyglycerin, mittlerer Polymerisationsgrad 3, Zusammensetzung:

16 % Mono-, 25 % Di-, 24 % Tri-, 19 % Tetra-,
9 % Penta-, 7 % Hexa-Glycerin

M-Glucosid   = Alpha-Methylglucosid

PGS III   = Polyglucosylsorbitol, mittlerer Polymerisationsgrad 2 bis 3, 70 %ige waßrige Lösung

PGS II   = Polyglucosylsorbitol, mittlerer Polymerisationsgrad 12, 70 %ige waßrige Lösung

## Ansprüche

1. Kohlenhydrat-Fettsäureester mit verbesserter Farbe und Fließfähigkeit, gekennzeichnet durch einen Gehalt von 1 bis 15 Gew.-% Polyetherpolyol.

2. Produkte gemäß Anspruch 1, in denen das Polyetherpolyol ein Polyglycerin ist.

3. Verfahren zur Herstellung von Kohlenhydrat-Fettsäureestern mit verbesserter Farbe und Fließfähigkeit durch einstufige Umsetzung von Kohlenhydraten mit Fettsäuren bei 180 bis 250 °C unter Alkalikatalyse in Gegenwart eines Farbverbesserers und nachfolgende Bleichung, dadurch gekennzeichnet, daß man dem Reaktionsgemisch 1 bis 15 Gew.-% Polyetherpolyol, bezogen auf die Gesamtmenge der eingesetzten Reaktanten, zusetzt, als Farbverbesserer ein Hypophosphit verwendet und die Bleichung mit Wasserstoffperoxid durchführt.

4. Verfahren nach Anspruch 3, dadurch gekennzeichnet, daß das Polyetherpolyol ein Polyglycerin mit einem mittleren Polymerisationsgrad von 2 bis 5 ist.

5. Verfahren nach Anspruch 4, dadurch gekennzeichnet, daß 4 bis 10 Gew.-% Polyglycerin, bezogen auf die Kohlenhydrate, eingesezt werden.

6. Verfahren nach Anspruch 3, dadurch gekennzeichnet, daß man die Umsetzung bei 190 bis 220 °C durchführt.

7. Kohlenhydrat-Fettsäureester, hergestellt nach einem Verfahren entsprechend den Ansprüchen 3 bis 6.

5